# EUROPEAN PATENT APPLICATION

(11) **EP 3 479 783 A1**
(43) Date of publication of application: **08.05.2019**
(21) Application number: 17823517.2
(22) Date of filing: 20.06.2017
(51) Int. Cl.: A61B 17/32, A61B 17/16

(54) **PIEZOSURGERY TOOL BIT**

(30) Priority: 04.07.2016 CN 201610518546
(71) Applicant: Beijing SMTP Technology Co., Ltd., Beijing 100083 (CN)
(72) Inventor: SUN, Yu, Beijing 100083 (CN); FENG, Zhen, Beijing 100083 (CN); LIU, Qingming, Beijing 100083 (CN); CAO, Qun, Beijing 100083 (CN)
(74) Representative: Balder IP Law, S.L.
(86) International application number: PCT/CN2017/089194
(87) International publication number: WO 2018/006705

(57) **Abstract**

Disclosed is a tool bit for an ultrasonic osteotome comprising a cutting portion (1) at a front end of the tool bit for an ultrasonic osteotome, a bit body (3) connected to a transducer and an arbor (2) connecting the cutting portion (1) and the bit body (3). The cutting portion (1) assumes a shape of a long cylinder, a front end of which is shrunk, and a plurality of spiral cutting edges (4) are formed on a side surface of the cylinder. When an operation is performed by using the tool bit for an ultrasonic osteotome of the present invention, the tool bit does not rotate, and thus will not cause damage to a body tissue such as pulling and tangling on the tissue. The tool bit for an ultrasonic osteotome of the present invention can be accurately positioned, and there is no slippage and deviation on a bone surface, so that the cutting process is stable and smooth and the maneuverability is high. The tool bit for an ultrasonic osteotome of the invention can be used for grinding, drilling, scraping, finishing, and other operations. It integrates drilling and grinding functions in one body, realizing a multi-purpose scalpel, reducing operating time and fatigue of a doctor.

## Description

### TECHNICAL FIELD

The present disclosure relates to the field of medical instruments and devices, in particular to a scalpel, and more particularly to a tool bit for an ultrasonic osteotome.

### BACKGROUND OF THE INVENTION

In modern society, with the development of medical technology, orthopedic surgery shows a trend of diversity. Accordingly, when performing a surgery, it is necessary to use different tool bits for a scalpel to perform cutting, grinding, scraping, clamping and other operations on an affected part of a patient according to different orthopedic conditions of disease.

In an orthopedic surgery, it is often necessary to grind and drill a bone, typically using a pneumatic or an electric drill device characterized by a high-speed rotation to cut a bone tissue to achieve a purpose of grinding and drilling a hole. In practical applications, on the one hand, the high-speed rotation of the drill bit will cause damages such as entangling and pulling of the surrounding tissues and organs, such as vessels, nerves and the like, which most need to be protected, and may cause fatal injury to a patient and thus lead to a surgical failure. On the other hand, a high-speed rotating drill bit will generate vibrations when it contacts a body tissue and thus it is easy to deviate from a specified position, that is, the operation process is not stable enough, and slippage and deviation may occur. This can cause damage to other non-surgical sites and cause danger. This also puts higher requirements on the level of operation of the medical staff, reduces the success rate of the surgery, and increases the risk of surgery for the patient.

### SUMMARY OF THE INVENTION

In view of the above problems in the prior art, the present disclosure provides a tool bit for an ultrasonic osteotome comprising a cutting portion at a front end of the tool bit for an ultrasonic osteotome, a bit body connected to a transducer, an arbor connecting the cutting portion and the bit body; the cutting portion assumes a shape of a long cylinder, a front end of which is shrunk, and a plurality of spiral cutting edges are formed on a side surface of the cylinder.

The tool bit for an ultrasonic osteotome of the invention generates high frequency vibration in a longitudinal direction, and can drill a small hole in a bone surface. As the tool bit advances in depth, the cutting edges grinds and scrapes an inner wall of the small hole to continuously expand a diameter of the hole. When this operation is repeated, an intended purpose of operation can be achieved.

In the tool bit for an ultrasonic osteotome according to an embodiment of the present invention, preferably, the tool bit for an ultrasonic osteotome has a hollow structure.

In the tool bit for an ultrasonic osteotome according to an embodiment of the present invention, it is preferable that the cutting edges are arranged at equal intervals on the side surface of the cylinder of the cutting portion.

In the tool bit for an ultrasonic osteotome according to an embodiment of the present invention, preferably, the arbor is bent at an angle.

In the tool bit for an ultrasonic osteotome according to an embodiment of the present invention, preferably, the bit body possesses a cylindrical shape, and the arbor has a cylindrical shape. An outer diameter of the arbor is smaller than an outer diameter of the bit body, and there is a smooth transition between the arbor and the bit body. An outer diameter of the cutter portion is smaller than an outer diameter of the arbor, and there is a smooth transition between the cutting portion and the arbor.

In the tool bit for an ultrasonic osteotome according to an embodiment of the present invention, it is preferable that the plurality of cutting edges are formed with teeth thereon.

In the tool bit for an ultrasonic osteotome according to an embodiment of the present invention, it is preferable that the teeth are arranged to be aligned in a circumferential direction.

In the tool bit for an ultrasonic osteotome according to an embodiment of the present invention, it is preferable that the teeth are arranged in a spiral pattern in a circumferential direction.

In the tool bit for an ultrasonic osteotome according to an embodiment of the present invention, preferably, the teeth starts from a position in the middle of the cutting portion near the arbor and ends at a distal end of the cylinder of the cutting portion.

In the tool bit for an ultrasonic osteotome according to an embodiment of the present invention, preferably, the bit body is provided with clamping faces for clamping.

The tool bit for an ultrasonic osteotome of the invention is exquisite and compact, and can accurately control a cutting amount and shape of a bone, reducing an amount of bone loss during an operation, accelerating a recovery time of a patient. In the process of osteotomy, a thermal effect of the tool bit for an ultrasonic osteotome according to the present invention can also play a role for hemostasis and coagulation and can reduce an amount of intraoperative blood loss. When an operation is performed by using the tool bit for an ultrasonic osteotome according to the present invention, the tool bit does not rotate, and thus does not cause damage to a body tissue due to pulling and tangling of the tissue. Meanwhile, the tool bit for an ultrasonic osteotome according to the present invention can be accurately positioned with no slippage and deviation, so that the cutting process is stable and smooth and the maneuverability is high. The tool bit for an ultrasonic osteotome of the invention can be used for grinding, drilling, scraping, finishing, and other operations. Moreover, it integrates drilling and grinding functions in one body, so that both a top end and a side surface of the cutting portion can perform operation on a bone tissue.

### BRIEF DESCRIPTION OF THE DRAWINGS

In order to more clearly illustrate the technical solutions in specific embodiments of the present invention or in the prior art, the drawings used in the description of the specific embodiments or the prior art will be briefly described below. Apparently, the drawings described in the following description are only some embodiments of the present invention, and those skilled in the art can obtain other drawings based on these drawings without creative work.
Fig. 1 is a perspective view of a tool bit for an ultrasonic osteotome according to a first embodiment of the present invention;
Fig. 2 is a front elevational view of a tool bit for an ultrasonic osteotome according to a second embodiment of the present invention;
Fig. 3 is a perspective view of a tool bit for an ultrasonic osteotome according to a third embodiment of the present invention;
Fig. 4A is a schematic view showing an embodiment of a cutting portion of a tool bit for an ultrasonic osteotome according to the present invention, with the cutting portion is provided with cutter teeth;
Fig. 4B is a schematic view showing another embodiment of a cutting portion of a tool bit for an ultrasonic osteotome according to the present invention, with the cutting portion is provided with cutter teeth;
Fig. 5(a) is a schematic cross-sectional view showing a first embodiment of a cutting portion of a tool bit for an ultrasonic osteotome according to the present invention;
Fig. 5(b) is a schematic cross-sectional view showing a second embodiment of a cutting portion of a tool bit for an ultrasonic osteotome according to the present invention;
Fig. 5(c) is a schematic cross-sectional view showing a third embodiment of a cutting portion of a tool bit for an ultrasonic osteotome according to the present invention;
Fig. 5(d) is a schematic cross-sectional view showing a fourth embodiment of a cutting portion of a tool bit for an ultrasonic osteotome according to the present invention;
Fig. 5(e) is a schematic cross-sectional view showing a fifth embodiment of a cutting portion of a tool bit for an ultrasonic osteotome according to the present invention; and
Fig. 5(f) is a schematic cross sectional view showing a sixth embodiment of a cutting portion of a tool bit for an ultrasonic osteotome according to the present invention.

### Reference numerals:

1-cutting portion; 2-arbor; 3-bit body; 4-cutting edge;
5-cutter teeth; 6-clamping face; 7-connection mechanism.

### DETAILED DESCRIPTION OF THE INVENTION

Embodiments of the present disclosure will be described hereinafter clearly and completely with reference to accompanying drawings. Apparently, the embodiments described herein are only portions of embodiments of the invention, rather than all embodiments of the invention. It is intended that all other embodiments obtained by those skilled in the art based on the disclosed embodiments without inventive labor are within the scope of the present invention.

In the description of the present disclosure, it is to be noted that the terms of 'center_, 'upper_, 'lower_, 'left_, 'right_, 'vertical_, 'horizontal_, 'internal_, 'external_, and the like simply indicate orientational or positional relationship based on the accompanying drawings and are used only for the purpose of facilitating and simplifying the description of the invention, rather than specifying or implying that any device or elements indicated must have a certain orientation, constitute with a certain orientation, or operate in a certain orientation. Therefore, these terms will not be interpreted as limiting the present invention. Further, the terms of 'first_, "second, and 'third_ are only used for description purpose, rather than being interpreted as specifying or implying relative importance.

In the description of the present disclosure, it is to be noted that, unless otherwise specified or defined clearly, the terms of 'attach_, 'connect to_, 'connect with_, 'couple_ and the like should be interpreted broadly. For example, they may refer to fixed connection, or detachable connection, or integral connection; they may refer to mechanical connection, or electrical connection; they may refer to direct connection, or indirect connection through an intermediate media, or internal communication between two components. For those skilled in the art, the specific meaning of these terms in the present disclosure may be understood in combination with specific situations.

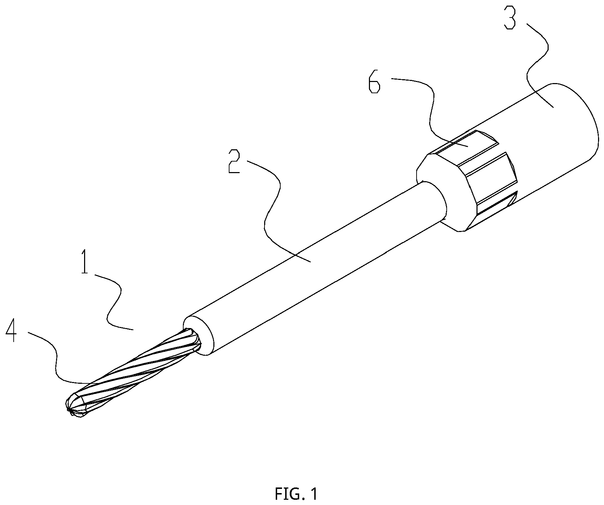

Fig. 1 is a perspective view of a tool bit for an ultrasonic osteotome according to a first embodiment of the present invention. As shown in Fig. 1, the tool bit for an ultrasonic osteotome according to the first embodiment of the present invention comprises a cutting portion 1 at a front end of the tool bit for an ultrasonic osteotome, a bit body 3 connected to a transducer, and an arbor 2 with one end connected to the cutting portion 1 and the other end connected to the bit body 3. The cutting portion 1 assumes a shape of a long cylinder, a front end thereof is shrunk, and a plurality of spiral cutting edges 4 are formed on a side surface of the cylinder. The tool bit for an ultrasonic osteotome of the invention uses ultrasound as power to generate micron-level vibration in a longitudinal direction, an operator can complete drilling and grinding and other operations on a bone with the tool bit for an ultrasonic osteotome without rotation, and no damage to a body tissue will be incurred by tangling, pulling and the like on the tissue. The operator can easily and accurately operate at a specified position by using the tool bit for an ultrasonic osteotome according to the present invention, and the tool bit for an ultrasonic osteotome will not slip or deviate. The tool bit for an ultrasonic osteotome of the invention has high safety, strong maneuverability and is simple for use, and can realize integrated operation of drilling and grinding. The top end as well as the cutting edges 4 on side surfaces of the tool bit for an ultrasonic osteotome all can operate on a bone tissue.

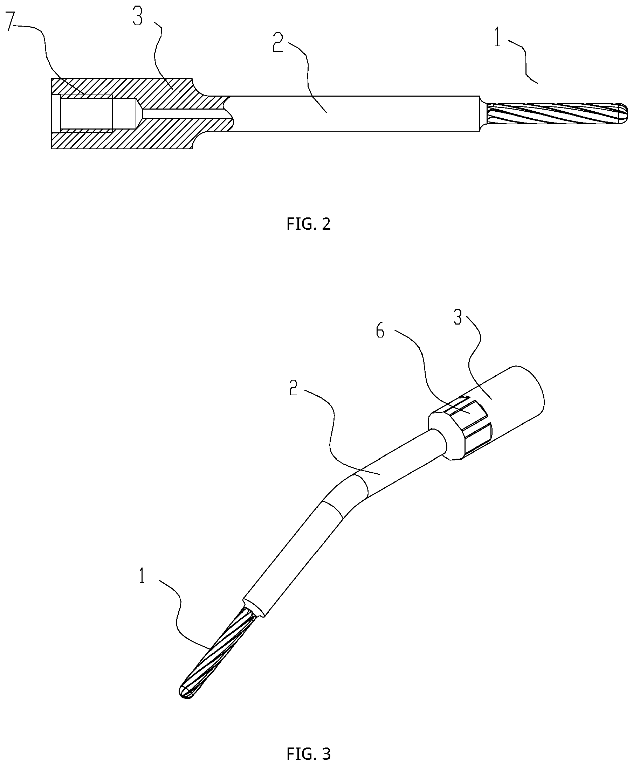

Fig. 2 is a front elevational view of a tool bit for an ultrasonic osteotome according to a second embodiment of the present invention. As shown in Fig. 2, the tool bit for an ultrasonic osteotome according to the second embodiment of the present invention is substantially similar to the tool bit for an ultrasonic osteotome of the first embodiment of the present invention in structure, except that the tool bit for an ultrasonic osteotome of the second embodiment of the present invention has a hollow structure. On the one hand, the tool bit for an ultrasonic osteotome with a hollow structure can save material, making the structure of the tool bit for an ultrasonic osteotome lighter, more labor-saving for gripping and more convenient for operation. On the other hand, the hollow structure can allow a fluid to flow therein so that a liquid can be passed therethrough to cool the tool bit for an ultrasonic osteotome or flush a wound, or play other fluid guiding roles.

In the tool bit for an ultrasonic osteotome according to the first embodiment and the second embodiment of the present invention, it is preferable that the cutting edges 4 are arranged at equal intervals on the cylindrical side surface of the cutting portion 1, thereby facilitating machining of the cutting edge 4 in identical intervals.

Fig. 3 is a perspective view of a tool bit for an ultrasonic osteotome according to a third embodiment of the present invention. As shown in Fig. 3, the tool bit for an ultrasonic osteotome according to the third embodiment of the present invention is substantially similar to the structure of the tool bit for an ultrasonic osteotome according to the first embodiment of the present invention, except that in the tool bit for an ultrasonic osteotome of the third embodiment of the present invention, an arbor 2 is bent at an certain angle. The tool bit for an ultrasonic osteotome with a curved arbor 2 can take a detour to arrive at the rear of a body tissue to perform an operation on an underlying bone, thereby expanding a range of application of the tool bit for an ultrasonic osteotome according to the present invention.

In the tool bit for an ultrasonic osteotome according to an embodiment of the present invention, as shown in Fig. 1 and Fig. 2, it is preferable that the bit body 3 has a cylindrical shape, and the arbor 2 has a cylindrical shape. An outer diameter of the arbor 2 is smaller than an outer diameter of the bit body 3, and there is a smooth transition between the arbor 2 and the bit body 3. An outer diameter of the cutting portion 1 is smaller than an outer diameter of the arbor 2, and there is a smooth transition between the cutting portion 1 and the arbor 2. The smooth transition structure effectively prevents stress concentration while preventing sharp edges and corners from injuring an operator.

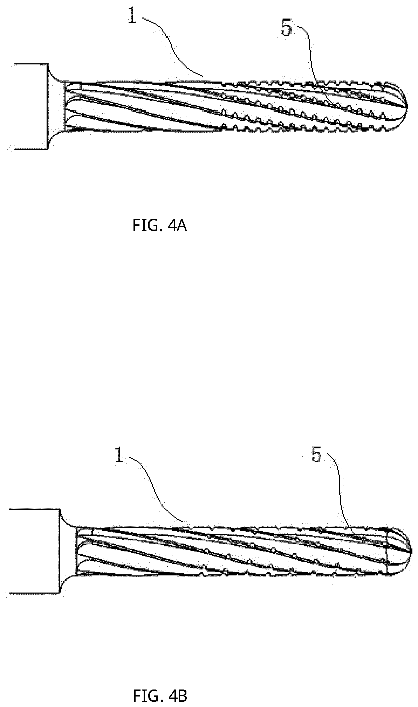

Fig. 4A is a schematic view showing an embodiment of a cutting portion formed with cutter teeth in a tool bit for an ultrasonic osteotome according to the present invention. Fig. 4B is a schematic view showing another embodiment of a cutting portion formed with cutter teeth in a tool bit for an ultrasonic osteotome according to the present invention. As shown in Figs. 4A and 4B, in the tool bit for an ultrasonic osteotome according to the present invention, the plurality of cutting edges 4 may be formed with cutter teeth 5 thereon. In the embodiment shown in Fig. 4A, the teeth 5 are arranged to be aligned in a circumferential direction. In another embodiment shown in Fig. 4B, the teeth 5 are helically arranged in a circumferential direction. Of course, the teeth 5 can also be arranged in any other form as long as they can perform a cutting operation on a body tissue.

As shown in Figs. 4A and 4B, in the tool bit for an ultrasonic osteotome according to the present invention, the cutter teeth 5 may start from a position in the middle of the cutting portion 1 near the arbor 2, and terminate at a distal end of the cylinder of the cutting portion 1. The portion of the cutting portion 1 at which the teeth 5 are provided is a main working portion of the cutting portion 1, thus the teeth 5 is provided at above position can greatly improve the working efficiency of the tool bit for an ultrasonic osteotome according to the present invention.

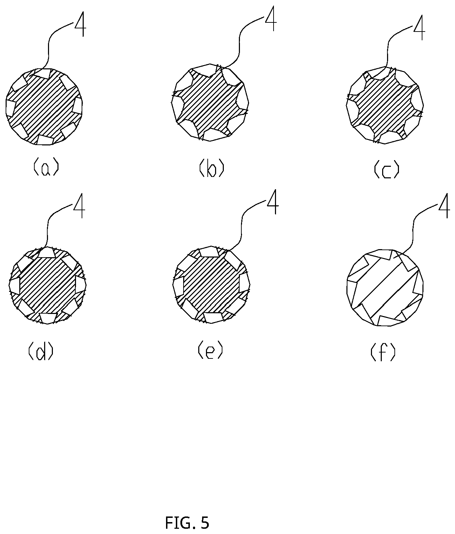

Fig. 5(a) to 5(f) are cross-sectional schematic views showing a first to sixth embodiments of a cutting portion 1 of a tool bit for an ultrasonic osteotome of the present invention, in which the cutting edges 4 are shown to have six different shapes. In the first embodiment of a cutting portion of a tool bit for an ultrasonic osteotome of the present invention shown in Fig. 5(a), the cutting edges 4 are formed by adjacent grooves, and a section (i.e., cross section), in a direction perpendicular to an axis of the tool bit, of the adjacent grooves is a part of a rectangle. In a second embodiment of a cutting portion of a tool bit for an ultrasonic osteotome of the present invention shown in Fig. 5(b), the cutting edges 4 are formed by adjacent grooves, and a section (i.e., cross section), in a direction perpendicular to an axis of the tool bit, of the adjacent grooves is a part of an irregular polygonal rectangle, and the formed cutting edges 4 are inclined clockwise or counterclockwise in the circumferential direction of the tool bit. In a third embodiment of a cutting portion of a tool bit for an ultrasonic osteotome of the present invention shown in Fig. 5 (c), the cutting edges 4 are formed by adjacent grooves, and also, a section (i.e., cross section), in a direction perpendicular to an axis of the tool bit, of the adjacent grooves is a part of an irregular polygonal rectangle, but it is different from the second embodiment of the cutting portion of the tool bit for an ultrasonic osteotome of the present invention in that, the cutting edges 4 formed in the third embodiment extend in a radial direction of the tool bit to form a radiation pattern. In a fourth embodiment of a cutting portion of a tool bit for an ultrasonic osteotome of the present invention shown in Fig. 5(d), the cutting edges 4 are formed by adjacent grooves, and a section (i.e., cross section), in a direction perpendicular to an axis of the tool bit, of the adjacent grooves is a part of a trapezoid, so that the formed cutting edges 4 have an inverted trapezoidal cross section and extend in a radial direction of the tool bit to form a radiation pattern. In a fifth embodiment of a cutting portion of a tool bit for an ultrasonic osteotome of the present invention shown in Fig. 5(e), the cutting edges 4 are formed by adjacent grooves, and a section (i.e., cross section), in a direction perpendicular to an axis of the tool bit, of the adjacent grooves is a part of a parallelogram, so that the formed cutting edges 4 have an inverted trapezoidal cross section and are inclined clockwise or counterclockwise in the circumferential direction of the tool bit In a sixth embodiment of a cutting portion of a tool bit for an ultrasonic osteotome of the present invention shown in Fig. 5(f), the cutting edges 4 are formed by adjacent grooves, and a section (i.e., cross section), in a direction perpendicular to an axis of the tool bit, of the adjacent grooves is a part of a right-angled triangle, so that the formed cutting edges 4 have a trapezoidal cross section and are inclined clockwise or counter clockwise in the circumferential direction of the tool bit. Of course, the cross-sectional shape of the cutting portion 1 is not limited to that shown in Figs. 5 (a) to (f), and any shape that can achieve bone scraping, bone cutting, and bone drilling can be used as a shape of the cutting edges 4.

As shown in Figs. 1 and 3, in the tool bit for an ultrasonic osteotome according to an embodiment of the present invention, the bit body 3 is provided with clamping faces 6 for clamping. It is convenient for an operator to clamp the clamping faces 6 with a clamping tool and screw the tool bit for an ultrasonic osteotome of the present invention onto the transducer.

As shown in Fig. 2, in the tool bit for an ultrasonic osteotome according to an embodiment of the present invention, the bit body 3 is provided with a connection mechanism 7. Preferably, the connection mechanism 7 is a threaded structure, which may be an external thread or an internal thread. Of course, the connection mechanism 7 is not limited to the threaded structure. Alternately, any connection mechanism 7 can be provided on the bit body 3 of the tool bit for an ultrasonic osteotome of the present invention, as long as it can fix the tool bit for an ultrasonic osteotome of the present invention to a transducer in a detachable manner.

Compared with the prior art, the tool bit for an ultrasonic osteotome of the invention is exquisite and compact, can accurately control a cutting amount and a shape of a bone, reduce an amount of bone loss during an osteotomy operation, and accelerate a recovery time of a patient. The tool bit for an ultrasonic osteotome according to the present invention has a hemostatic effect and a blood coagulation effect during a process of cutting a bone, reducing an amount of intraoperative blood loss. When the tool bit for an ultrasonic osteotome according to the present invention is used for an operation, the tool bit will not rotate, and it will not cause damage to a body tissue due to tangling, pulling, etc. on the tissue, and thus possesses high safety. At the same time, the tool bit for an ultrasonic osteotome of the invention can be positioned accurately during an operation with no slippage and deviation, and has a stable and smooth cutting process and a high maneuverability. The tool bit for an ultrasonic osteotome of the invention can be used for grinding, drilling, scraping, finishing, etc., and integrates drilling and grinding functions in one body. Both the top end and the side surface of the cutting part 1 can be used to perform an operation on a bone tissue. The tool bit for an ultrasonic osteotome of the invention is simple in structure, convenient for machining, flexible for operation and has a wide range of application. When the tool bit for an ultrasonic osteotome of the invention is used for grinding a bone, it has high efficiency, fast speed and short operating time, which can alleviate pains suffered by a patient and reduce labor intensity of a doctor.

Finally, it should be noted that the above embodiments are only used to describe the solutions of the present invention, rather than limiting the present invention. Although detailed descriptions of the invention are made with reference to the above embodiments, it would be appreciated by those skilled in the art that various changes or modifications to the above embodiments may be made or equivalent substitutions to portion of or all features in those embodiments may be made. Such changes, modifications or substitutions will not make the spirit of the relevant solutions depart from the scope of the present invention.

## Claims

1. A tool bit for an ultrasonic osteotome, comprising a cutting portion at a front end of the tool bit for an ultrasonic osteotome, a bit body connected to a transducer, and an arbor connecting the cutting portion and the bit body, wherein
the cutting portion assumes a shape of a long cylinder, a front end of which is shrunk, and a plurality of spiral cutting edges are formed on a side surface of the cylinder.

2. The tool bit for an ultrasonic osteotome of claim 1, wherein
the tool bit for an ultrasonic osteotome has a hollow structure.

3. The tool bit for an ultrasonic osteotome of claim 1 or 2, wherein
the cutting edges are arranged in identical intervals on the side surface of the cylinder of the cutting portion.

4. The tool bit for an ultrasonic osteotome of claim 1 or 2, wherein
the arbor is bent at an angle.

5. The tool bit for an ultrasonic osteotome of claim 1 or 2, wherein
the bit body has a cylindrical shape, the arbor has a cylindrical shape;
an outer diameter of the arbor is smaller than an outer diameter of the bit body, and there is a smooth transition between the arbor and the bit body;
an outer diameter of the cutter portion is smaller than an outer diameter of the arbor, and there is a smooth transition between the cutting portion and the arbor.

6. The tool bit for an ultrasonic osteotome of claim 1 or 2, wherein
the plurality of cutting edges are formed with teeth thereon.

7. The tool bit for an ultrasonic osteotome of claim 6, wherein
the teeth are arranged to be aligned in a circumferential direction.

8. The tool bit for an ultrasonic osteotome of claim 6, wherein
the teeth are arranged in a spiral pattern in a circumferential direction.

9. The tool bit for an ultrasonic osteotome of claim 6, wherein
the teeth starts from a position in the middle of the cutting portion near the arbor and ends at a distal end of the cylinder of the cutting portion.

10. The tool bit for an ultrasonic osteotome of claim 1 or 2, wherein
the bit body is provided with clamping faces for clamping.
